# EUROPEAN PATENT APPLICATION

(11) **EP 1 457 183 A2**
(43) Date of publication of application: **15.09.2004**
(21) Application number: 04014441.2
(22) Date of filing: 23.09.1999
(51) Int. Cl.: A61F 13/00, D04H 13/00

(54) **A wound dressing**

(30) Priority: 28.09.1998 ZA 9808838
(62) Divisional of application: 99943167.9
(71) Applicant: Vibriant Technology Services Limited, London W1S 4HY (GB)
(72) Inventor: Mouton, Johannes Petrus, Club View Centurion 0157 (ZA)
(74) Representative: Waddington, Richard

(57) **Abstract**

A wound dressing (10) includes a first and second absorbent layer (12.2, 12.3), each absorbent layer being of a non-woven fabric of fibres, and each being able to absorb liquid and a screen (12.1) comprising polyester and cotton fibres between, and bonded to, the two absorbent layers (12.2, 12.3). The bonded absorbent layers (12.2, 12.3) and the screen (12.1) form essentially a single, layered fabric body. The bonding between the first and second absorbent layers (12.2, 12.3) and the screen (12.1) is brought about by a needle-punching process.

## Description

This invention relates to wound dressings.

According to the present invention there is provided an apparatus and method as set forth in the appended claims. Preferred features of the invention will be apparent from the dependent claims, and the description which follows.

According to a first aspect of the invention, there is provided a wound dressing comprising
a first and a second absorbent layer, each absorbent layer being of a non-woven fabric of fibres, and each being able to absorb liquid; and
a screen comprising polyester and cotton fibres between, and bonded to, the two absorbent layers, so that the two absorbent layers and the screen form essentially a single, layered fabric body, the bonding between the first and second absorbent layers and the screen being brought about by a needle-punching process in which the punching density is about 1700 - 1900 punches per minute.

By "punching density" is meant the number of times a needle perforates the absorbent layers and the screen per square cm in order to bond them together. This is typically conducted on a needleloom which has a needle board with about 30 000 needles and about 6 000 needles per linear metre, at a punching rate of about 300 punches per minute.

Preferably, the bonding between the first and second absorbent layers may be brought about by a needle-punching process in which the punching density is about 1800 punches per square cm.

The fibres may be porous fibres.

The wound dressing may include at least one liquid permeable layer which is substantially non-adherent to human or animal tissue overlaying, and bonded to, at least one of the absorbent layers.

The wound dressing may include a further liquid permeable layer disposed such that the single, layered fabric body is sandwiched between the two liquid permeable layers.

Each absorbent layer may be of porous polyester fibres. The screen may comprise 80% polyester and 20% cotton fibres. It may have a thread density of about (120 to 150 threads per square inch) 18.6 to 23.2 threads/cm². It may have a yarn count of about 32 to 40. It may have a weight per unit area of approximately 100 g/m².

The absorbent layers of non-woven fabric may be in the form of two fibre batts fabricated on a needleloom, each being made of 100% polyester fibre. The fibre batts may have a fineness of about 1.5 denier and a fibre length of about 7 - 8 cm, preferably about 7.62 cm (i.e. about 3 inches). They may have a weight per unit area of approximately 300 g/m².

The single, layered fabric body may have a mass of about 700 - 750 g/m². It may have a thickness of not more than about 3 mm.

The liquid permeable layer may be of a foraminous or perforated synthetic polymeric material. The synthetic polymeric material may be selected from "MYLAR" (trade name) and "TELFA" (trade name).

The bonding between the or each liquid permeable layer and the or each absorbent layer may be achieved by heat treatment under pressure.

According to another aspect of the invention there is provided a method of making a wound dressing, the method including the steps of
fabricating two needle-punched fibre batts of fibres on a needleloom;
fabricating a tightly woven screen of polyester and cotton fibres;
locating the screen between the needle-punched fibre batts to form a composite structure in which the screen forms an inner layer between the two fibre batts; and
needle-punching the composite structure on a needleloom to produce a single, layered fabric body in a needle-punching process in which the punching density is about 1700 - 1900 punches per square cm.

Preferably, the punching density will be about 1800 punches per square cm.

The method may include the further step of securing a liquid permeable layer, which is substantially non-adherent to human or animal tissue, to at least one side of the layered fabric body.

The needle-punched fibre batts may be of porous polyester. The screen may be fabricated of 80% polyester and 20% cotton yarns. It may be fabricated to have a thread density of about (120 to 150 threads per square inch) 18.6 to 23.2 threads/cm². It may be fabricated to have a yarn count of about 32 to 40. It may be fabricated to have a weight per unit area of approximately 100 g/m².

The composite structure may be needle-punched to produce a single layered fabric body having a mass of about 700 - 750 g/m². The needle punching process may be repeated according to the thickness or effect desired. The composite structure may be needle-punched so that the thickness of the single layered fabric body will typically be not more than about 3 mm.

The fibre batts may have a fineness of about 1.5 denier. They may be fabricated to have a weight per unit area of approximately 300 g/m².

The liquid permeable layer may be of foraminous or perforated synthetic polymeric material. It may be selected from "MYLAR" (trade name) and "TELFA" (trade name).

Securing the liquid permeable layer to the at least one side of the layered fabric body may be achieved by heat treatment under pressure.

The invention extends to a method of removing exudate from a wound, the method including the step of applying to an exudating wound at least one wound dressing as hereinbefore described.

The invention will now be described, by way of example, with reference to the following drawings, in which:
Figure 1 is a schematic, exploded three-dimensional view of a wound dressing in accordance with the invention;
Figure 2 is a schematic, exploded three-dimensional view of another embodiment of a wound dressing in accordance with the invention; and
Figure 3 is a schematic, exploded three-dimensional view of another embodiment of a wound dressing in accordance with the invention.

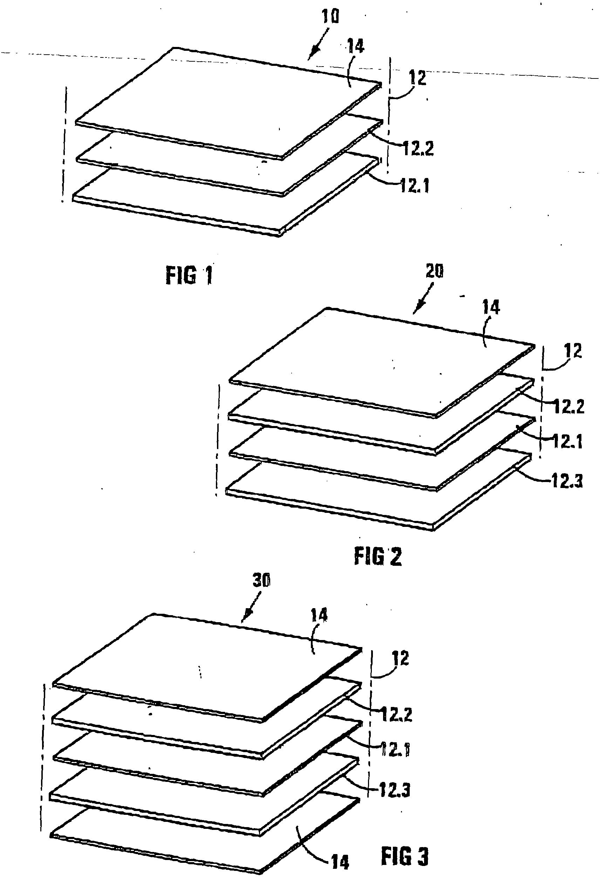

Referring to Figure 1, reference numeral 10 generally indicates a wound dressing in accordance with the invention.

The wound dressing 10 is shown in exploded view in the drawing. The dressing 10 includes a first absorbent layer in the form of a fibre batt 12.2 and a second absorbent layer in the form of a fibre batt 12.3. The fibre batts 12.2 and 12.3 are made of non-woven fabric comprising porous polyester fibres. The dressing 10 includes a tightly woven screen 12.1 comprising polyester and cotton fibres sandwiched between the two fibre batts 12.2 and 12.3. The screen 12.1 is of a woven fabric comprising 80% polyester and 20% cotton yarns and has a thread density of about (120 to 150 threads per square inch) 18.6 to 23.2 threads/cm², a yarn count of about 32 to 40 and a weight of about 100 g/m². The two absorbent layers 12.2 and 12.3 and the screen form essentially a single, layered fabric body, indicated in the drawing by reference numeral 12.

Referring to Figure 2, reference numeral 20 generally indicates another embodiment of a wound dressing in accordance with the invention.

The wound dressing 20 resembles the wound dressing 10 and the same numbers have been used to indicate the same or similar features of the dressings 20 and 10. The dressing 20 differs from the dressing 10 only in that an outer liquid permeable layer 14 is secured to the fibre batt 12.2 so that the wound dressing 20 comprises four separate layers rather than three as in the wound dressing 10.

Referring to Figure 3, reference numeral 30 generally indicates another embodiment of a wound dressing in accordance with the invention. The wound dressing 30 resembles the wound dressing 20 and the same numbers have been used to indicate the same or similar features of the dressings 30 and 20. The dressing 30 differs from the dressing 20 only in that a further outer liquid permeable layer 14 is secured to the fibre batt 12.3 so that the wound dressing 30 comprises five separate layers rather than four as in the wound dressing 20.

The liquid permeable layer 14 is bonded to the composite layer 12 by heat treatment under pressure. In a preferred embodiment of the invention the liquid permeable layers are of the material manufactured by Kendall (Pty) Limited and sold under the name "MYLAR" or the material manufactured by Macmed Healthcare Limited and sold under the trade name "TELFA".

The fibre batts 12.2 and 12.3 are fabricated on a needleloom. Each fibre batt 12.2, 12.3 is made of 100% polyester fibre, with a fineness of about 1.5 denier, a fibre length of about 7.62 cm (about 3 inches) and each has a weight of about 300 g/m². The two fibre batts 12.2 and 12.3 are needle-punched onto each side of the screen 12.1 thus making one completed fibre batt or single, layered fabric body with a middle screen 12.1 and which weighs about 700 g/cm². The thickness of the single, layered fabric body 12 is not more than about 3 mm.

The needle-punching process by which the fibre batts 12.2 and 12.3 and the screen 12.1 are bonded together is conducted on a needleloom which has 30 000 needles on the needle board and 6 000 needles per linear metre. The needle-punching is conducted at a punching rate of 300 punches per minute so that the resulting punching density is 1,800 punches per square cm. This process pushes fibres from the fibre batts 12.2 and 12.3 through the screen 12.1, thereby bonding the fibre batts 12.2 and 12.3 to the screen 12.1 and to each other.

In different embodiments, the needle-punching process is conducted once or several times, on either side depending on the thickness required. The average thickness of the completed fibre batt is not more than about 3 mm. The Applicant has found that, if the material is much thicker than 3 mm, absorption by the wound dressing is slower and the ability of the wound dressing to "hold and lift" exudate from a wound becomes less effective. Particularly, where a number of layers of the wound dressing of the invention are placed on, or in, a wound which is producing a large amount of fluid, and in which the lower layers become saturated, the Applicant has found that, if the wound dressing is thicker than about 3 mm, transfer of the fluid through successive layers of the wound dressing is less effective. Thus, in use, the wound dressing of the invention may be used in several layers. It may also be folded or rolled for insertion into a wound cavity.

A complete fibre batt with the middle screen as described above was tested by the South African Bureau of Standards (SABS). The test results are tabulated below:

**TABLE 1**

| | | | |
|---|---|---|---|
| Tests | Results -SABS samples No. R2455A & R2455 B | Requirements as stated in CKS 464:1993 (as amended) | Methods of test, CKS 464:1993 (as amended) subclause reference |
| Construction | The dressing consists of 3 layers and a woven inner layer sandwiched between the 2 outer layers. The dressing has been needle-punched to create adhesion between the 3 layers. | - | Visual Examination |
| Fibre composition, % Outer layers (non-woven fabric) Inner layer (woven fabric) | All polyester Cotton and polyester | Cotton, viscose or thermobonding fibres(e.g. polypropylene ) - | 6.5 . |
| Mass per unit area, g/m² | 745 | - | 6.6 Determined on the composite sample |
| Bursting strength, kPa | 3 819 | - | 6.7 Determined on the composite sample |
| Sterility | Does not comply | Sterile packed dressings shall be sterile | 6.15 |
| Fluorescence Non-woven Woven fabric | Occasional spots Fluorescent | Not more than occasional point of intense blue fluorescence | 6. 9 |
| Absorption rate, s | 1.1 | 10 max | 6.10 |
| Ash content, g/kg | 0,3 | 5 max | 6.11 |
| pH value of aqueous extract | 7 | 7 ± 2 | BS3266 cold water extraction |
| Freedom from dyes | No yellow, blue or green tint | Percolate may show a yellow colour but not green or blue tint | 6.14 |
| Steam sterilization | Complies | Shall not show any appreciable deterioration in handle | 6.16 |

Being of a different construction, the construction, the mass per unit area and the bursting strength results of samples No. R2455A and R2455B were not compared with the requirements of the above specification.

In Table 1 test samples No. R2455A and R2455B refer to test samples for properties specified in The South African Department of Trade and Industry specification: CKS 464 : 1993 (as amended) for non-woven surgical dressings published by the SABS.

The wound dressing of the invention is a dispersion, high-absorbent, low adherent, disposable wound dressing.

It is an advantage of the invention illustrated that the wound dressing can be used for a wide variety of wounds including high exudating wounds, deep cavity wounds, superficial wounds (with ointments), burn wounds (where the low adherence properties of the wound dressing of the invention are important), transudating wounds, abscesses, ulcers, diabetic foot wounds, external cancer wounds, non-healing wounds, sinus wounds, and the like in both humans and animals. It is a further advantage of the invention illustrated that the wound dressing can be used as a surgical drain. It is a unique feature of the invention that both body fluids and bacteria can be dispersed from, or carried away from, the inside of a wound or cavity by a number of the wound dressings of the invention to the dressings on the outside of the wound or cavity. This enables wounds to heal from the inside and not from the outside. There is no breakdown in the material of the wound dressing after it has made contact with bodily fluids.

Generally, non-adherent absorbent dressings are made of gauze and porous fibres, including natural fibres such as cotton and synthetic fibres such as rayon and combinations thereof. A problem associated with such absorbent dressings is that the porous fibres absorb moisture or fluid from the wound. This leads to a phenomenon known as "strike through". Such wound dressings also to a certain extent, depending upon the type of dressing, tend to adhere to an exudating or transudating wound. Removal of such dressings from the wound often results in reopening of the wound and damage to newly formed granulating tissue.

It is an advantage of the invention illustrated that the tightly woven screen, which forms a discreet layer within the non-woven batt, serves to cause the exudate absorbed by the non-woven fabric part of the dressing to be dispersed along the screen. This serves to prevent or inhibit "strike through" and results in transport of the fluid away from the site of the wound. The advantage of this is that both fluid and bacteria are carried away from the wound thereby encouraging healing of the wound. It is a further advantage of the invention illustrated that the liquid permeable layer does not produce lint, fluff or the like, which are often associated with prior art wound dressings of which the Applicant is aware, and essentially does not adhere to the wound. This reduces both the trauma associated with changing dressings particularly in the case of serious wounds and prevents or inhibits damage to the healing wound caused by the removal of the dressing.

It is a further advantage of the invention illustrated that the three layers comprising the wound dressing can be bonded together by a single needle-punching process. Prior art processes known to the Applicant generally involve more than one needle-punching process to bond different layers together.

Without being bound thereby, the Applicant believes that the needle-punching process by which the wound dressing of the invention is fabricated and which involves a punching density of about 1,800 per square cm results in the dispersion action inside the dressing which distinguishes it from prior art absorbent dressings. It is the unique dispersion action inside the dressing which enables the dressing to "hold and lift" exudate or body fluids. Because of the dispersion action of the wound dressing of the invention, even once a dressing is saturated, a successive layer of the wound dressing will cause the fluids to be drawn away from the site of the wound so that healing can take place because of the removal of the fluids and associated bacteria from the wound. The success of the wound dressing of the invention, as a result of the dispersion action of the dressing, appears to be brought about by the choice of fibres and the needle-punching procedure used to fabricate the dressing.

## Claims

1. A method of making a wound dressing including the steps of:
fabricating two needle-punched fibre batts of fibres on a needle loom;
fabricating a tightly woven screen of polyester and cotton fibres;
locating the screen between the needle-punched fibre batts to form a composite structure in which the screen forms an inner layer between the two fibre batts; and
needle punching the composite structure on a needle loom so that fibres are pushed from the two batts through the screen to bond the batts and the screen together to form a single, layered fabric body in which the screen is a discreet layer between the separate batts.

2. A method according to claim 1 wherein the bonding is achieved by use of a needle loom of the kind having about 30000 needles with about 6000 needles per linear metre which gives a punched density for the three separate layers of about 1700-1900,

3. Material made according to the method of claim 1 or 2.

4. Multiple layers of material according to claim 3 arranged to allow transfer of fluid through successive said layers.

5. A wound dressing made according to the method of claim 1 or claim 2.

6. A wound dressing comprising two needle-punched fibre batts, and a tightly woven screen of cotton and polyester fibres, wherein the screen is located between the needle-punched fibre batts to form a composite structure in which the screen forms an inner layer between the two fibre batts; wherein the composite structure has been needle-punched causing fibres to be pushed from the two batts through the screen to form a single, layered fabric body in which the screen is a discreet layer between the fibre batts.

7. The wound dressing of claim 6, which has a thickness of less than or equal to about 3mm.

8. The use of a wound dressing as claimed in any one of claims 5 to 7, as a surgical drain.

9. A method of making a wound dressing as claimed in any one of claims 5 to 7, which involves a single needle-punching process.

10. A method of making a wound dressing as claimed in claim 1, claim 2 or claim 9 in which the bonding between the two fibre batts and the screen is achieved by heat treatment under pressure.
